# EUROPEAN PATENT APPLICATION

(11) **EP 2 565 827 A1**
(43) Date of publication of application: **06.03.2013**
(21) Application number: 10850841.7
(22) Date of filing: 29.10.2010
(51) Int. Cl.: G06K 9/78, A61B 5/117

(54) **DEVICE FOR PRODUCING IMAGES OF IRISES OF THE EYES**

(30) Priority: 27.04.2010 RU 2010116537
(71) Applicant: Antonov, Dimitry Evgenievich, Moscow 125040 (RU)
(72) Inventor: Antonov, Dimitry Evgenievich, Moscow 125040 (RU)
(74) Representative: HOFFMANN EITLE
(86) International application number: PCT/RU2010/000638
(87) International publication number: WO 2011/136686

(57) **Abstract**

The device relates to the technology of protecting different objects from access by unauthorized personnel by means of identification of an individual on the basis of an image of the iris of the eye of said individual and can be used in the diagnosis of the state of organs and functional systems in an organism on the basis of the iris of the eye.

The device for producing images of irises of the human eyes is directed to simplifying the design, the use conditions and the possibility for simultaneous production of a plurality of sets of characteristic features, i.e. a plurality of codes, which makes it possible to increase the accuracy and speed of identification of an individual.

Said result is achieved in that the device for producing images of irises of the human eye comprises a means (1) for illuminating the iris of the eye in one or more spectral ranges which are close to the visible range, a means (2) for recording the signal reflected from the eyes and a means (4) for processing the images produced, said means comprising a unit (5) for detecting the centres of the pupils of the eyes on the image, a unit (6) for determining coordinates of the eyes relative to a set centre of the display and for constructing a straight reference line connecting the centres of the pupils, a unit (7) for processing the images of the iris of the eye in a normalized coordinate grid with segmentation and isolation of information fields, a unit (8) for generating codes, and a unit (9) for storing said codes.

## Description

The present invention relates to the technology of protecting different objects from access by unauthorized personnel by means of identification of an individual on the basis of an image of the iris of the eye of said individual and can be used in the diagnosis of the state of organs and functional systems in an organism on the basis of the iris of the eye.

The various techniques and devices are known intended for identification of an individual by a complex of specific, peculiar to him features, available on the iris of the eye (see US 4641349).

The device is known designed for identification of a person by his iris of the eye, comprising a means for registering the iris in the form of a video camera with a lens, connected to the obtained information processing unit (see JP 10137223). The information processing unit is connected to the transmission means designed for transmitting the information to the individual, whose personality is to be identified. Said transmission means is accomplished in the form of a loudspeaker, which informs the person being identified about required changing of his position to obtain an optimal for recognition iris of the eye image. A disadvantage of the known system is its relative complexity, consisting in the fact that the system of obtaining information on the basis of the iris of the eye should perform its relatively fast analysis, reveal the shortcomings (e.g., a lack of image definition), and with a relatively intricate announcing system inform an individual being identified on necessity of some movements relative to the iris of the eye stabilizing means.

The device is known for identifying an individual by his iris of the eye, including the iris of the eye registration means in the form of a TV camera, a means for illuminating the iris of the eye of an individual being identified, using semiconductor emission source (see JP 10137220), and a means for creating a database on the basis of the iris of the eyes, which subsequently is used for identification of individuals. Therewith, in the process of registering image of the iris, the eye is illuminated by a narrow spectral range optical radiation. Each image before being loaded into the database undergoes processing in the computer with the help of special software and is stored in the form of a certain code. To realize the process of identification of an individual his iris of the eye image is recorded and stored, and the fixed image is processed and compared with the images stored in the database.

The shortcoming of the known device for an individual identification lies in its relatively low accuracy, because for identification of an individual only one set of characteristic features (i.e., only one code) is used.

The closest to the claimed device is the known device for identification of an individual on the basis of his iris of the eye image, containing an image recording means. It was found that the use of illumination of one spectral range, due to the differences in coloring of image of the eye pigment, in some cases does not allow to determine accurately the boundary between the white and the iris of the eye; besides, a number of the characteristic features of the iris of the eye can not be disclosed because of a low contrast when the illumination is used, since the illumination wavelength is close to the color of these elements. That is why in the said device there are several illuminating elements are provided with various emitted radiation wavelengths. At first, the eye of a person being identified is illuminated with a light close to IR, the iris pigment color is detected, and, depending on the result, the optical emission source wavelength is selected, which will be used for illumination of the iris at the moment of recording its image. As a result of processing the image, the corresponding code is formed which is stored in the database and used for comparing with a current code value generated upon re-registration of the iris image of a person being identified (see JP 2006031185).

The disadvantage of the known individual identification device, in spite of the fact, that obtained codes contain information on more characteristic features, is its relatively low accuracy, since only one set of characteristic features (i.e., only one code) is used for identification of an individual. Besides, the device has a relatively complicated design, is difficult in operation, and requires quite long time for obtaining the iris images due to necessity of selection of the optimal radiation wavelength to obtain a high-quality image required for identification. The process of generating the code, storing it in the database, and identification of an individual by way of comparing the re-generated code with the stored one also requires quite a long time and can result in mistakes due to the fact a person being identified at the moment of stabilization of the required for it images may incline the head, which results in the fact, that the image obtained in such conditions may coincide with the iris images of the other, previously identified persons, obtained at the "standard" position of the face in field of vision of the recording device. Moreover, while forming the identification code a head inclination angle (and, correspondingly, the iris angle of turn in certain limits) is not taken into account due to the physically ambiguous approach of a person being identified to the field of vision of the iris image stabilization means, which significantly increases an identification time because of shuffling the head inclination angles values of the re-generated iris image with simultaneous comparing with the values stored in the database.

Claimed as a utility model the present device for producing images of irises of the human eyes is directed to simplifying the design, the use conditions and the possibility for simultaneous production of a plurality of sets of characteristic features, i.e. a plurality of codes, which makes it possible to increase the accuracy and speed of identification of an individual.

Said result is achieved, in that the device for producing images of irises of the human eye comprises a means for illuminating the iris or the eye in one or more spectral ranges which are close to the visible range, a means for recording the signal reflected from the eyes and a means for processing the images produced, said means comprising a unit 5 for detecting the centres of the pupils of the eyes on the image, a unit 6 for determining coordinates of the eyes relative to a set centre of the display and for constructing a straight reference line connecting the centres of the pupils, a unit for processing the images of the iris of the eye in a normalised coordinate grid with segmentation and isolation of information fields, a unit for generating codes, and a unit for storing said codes.

Equipment of the device with the means for iris illumination in one or more spectral ranges close to the visible range allows to obtain several images of the same iris of the eye, in which the characteristic points (image-making elements) will have different locations in the same be differently located in the same coordinate grid system. Therewith, the characteristic features that have low contrast in one color spectrum and/or in the case of being illuminated with a one wavelength radiation, and may be omitted by the image processing and recognition system, will be more contrast in another color spectrum and/or when illuminated by the corresponding other wavelength radiation. This, in turn, allows forming the unique identification codes for each of the images, and each code is strictly individual and belonging only to that person. Therefore, the identification, carried out by several codes simultaneously, greatly increases the accuracy of the identification, because the person being identified is considered to be definitely established when coincide all codes obtained by processing several images.

Use in the iris registration process of illumination with the optical radiation, spectral range of which is close to the visible light, improves the identification accuracy, since, on the one hand, allows to receive the iris color image practically without distortion of the spectral characteristics of its elements, and, on the other hand, to select sufficiently high number of the spectra (or spectral lines, or spectral ranges), in which processing of the image can be performed.

Providing of the device with a means for recording the signal reflected from the eyes allows for stabilizing the iris image for subsequent processing and storing of the images obtained.

Providing the device with the obtained images processing means is necessary in order to generate identification codes on the basis of said images.

Providing the obtained image processing means with a unit for detecting the centres of the pupils of the eyes on the image and with a unit for determining coordinates of the eyes relative to a set centre of the display and for constructing a straight reference line connecting the centres of the pupils allows essentially accelerate both generating codes for storing and their comparing with the codes of the person being identified at the moment, as well as to improve identification accuracy, even in the case if only one wavelength (monochromatic) radiation is used for illumination. Indeed, if we take into account that the person being identified at the moment of stabilizing the images, required for the process, can incline the head, as a result of which the image obtained in such conditions may coincide with the iris images of the other, previously identified persons, obtained at the "standard" position of the face in the field of vision of the recording device, construction of the straight reference line connecting the centres of the pupils allows to define (restore) a "standard" position of the face, and thus, to avoid the errors. Accordingly, construction of the straight reference line connecting the centers of the pupils allows using it as a basis for forming a, normalized grid, in which location of the characteristic elements of the iris is calculated and the identification codes are formed. Performing calculation in the normalized coordinate grid reduces the identification codes formation time.

Any known monochromatic or close to them (with a narrow spectral range) incandescent light sources with corresponding optical filters, LEDs, etc., may be used as illumination means.

Any available prior art devices (for instance, video cameras, digital cameras, etc.) may be used as image recording means, but for the purpose of easy digitalizing and processing (analyzing, encrypting, recognizing) the recorded images with the computer, it is preferable to use CCD or CMOS matrices as image recording means.

As a recorded image processing means, including a unit for detecting pupil centres on the image, a unit for determining coordinates of the eyes relative to a set centre of the display and for constructing a straight reference line connecting the pupil centres, a unit for processing the images of the iris of the eye in a normalized coordinate grid with segmentation and isolation of information fields, a unit for generating codes and a unit for storing said codes, a personal computer with a corresponding software can be used. Said software may comprise a set of programs, such as: a program of determining coordinates of the eyes relative to a set centre of the display; a program of constructing a straight reference line connecting the pupil centres; a program of processing the images of the iris of the eye in a normalized coordinate grid with segmentation and isolation of information fields; a code-generating program; a database for storing said codes.

The essence of the claimed device is illustrated by an its embodiment example and by a figure showing in general form the basic schematic diagram of the device for recording images of irises of the eyes.

Device for producing images of irises of the eyes, in the most general case, comprises: a means 1 for illuminating the iris of the eye in one or more spectral ranges which are close to the visible range, a means 2 for recording the signal reflected from the eyes, located in a field of view of the optical projection system 3, building an image of the iris of the eye being registered in the plane of photosensitive elements of CCD matrix, and a means 4 for processing the images produced. Means 1 for illuminating the iris of the eye, means 2 for recording the signal reflected from the eyes and the optical projection system 3 can be placed in any common case (not shown).

The recorded image processing means 4, for which, any personal computer can be used, includes unit 5 for detecting pupil centres on the image, unit 6 for determining coordinates of the eyes relative to a set centre of the display and for constructing a straight reference line connecting the pupil centres, unit 7 for processing the images of the iris of the eye in a normalized coordinate grid with segmentation and isolation of information fields, unit 8 for generating codes and unit 9 for storing said codes.

The device operates in the following way. A person to be identified is placed in a field of view of optical projection system 3, which renders an image of iris of the eyes to be registered in the plane of photosensitive elements of recording means (CCD matrix) 2, capable of producing a high-quality image of iris of the eyes. Herewith, in the process of recording the image of the iris of the eyes, it is illuminated with optical radiation from sources 1 with different spectral ranges and wavelengths. For this purpose various well known, radiation sources can be used, such as LEDs of a curtain emission range, or some other sources. The resulting image is converted to digital form and transmitted to means 4 for processing the images produced (a personal computed with appropriate software). In accordance with the image processing software, unit 5 for detecting pupil centres on the image calculates location of the centers of the pupils of the eyes, and then unit 6 determines coordinates of the eyes relative to a set centre of the display and creates a straight reference line connecting the pupil centers of the eyes. As a set center of display, for instance, the left pupil center can be selected. Then the X-axis is oriented along a straight line connecting the pupil centers of the eyes and is directed, toward the right eye. Alternatively, the middle of the line, connecting the pupil centers of the eyes, can be selected as a reference center. The X-axis is oriented along the above-mentioned line.

Unit 7 for processing the image of the iris of the eye in a normalized coordinate grid selects information fields, and then, improves their quality by means of digital filtration algorithms and converses useful information to suitable for comparison form. At the stage of information field selection a search of visual clutter (eyelashes, eyelids and glare) in the frame is executed. Informational field of the eye is the whole area of the iris, except for the area occupied by a visual clutter. Digital filtration is needed to reduce noise in the frame and to detect color intensity variations in the various spectral bands. At the stage of conversion of the prepared image to the code, the image is compressed to a data set (code), so that it becomes possible to introduce a measure of similarity of said codes in order of identification, or verification of the person's identity.

On the basis of isolation and analysts of the images the irises of the eyes, the codes for each of them is formed, which are stored in a database as the reference codes. Later, when there is a problem of identification of a person to permit access or passage, for example, to a guarded zone, some deposit, while crossing the borders of states, the described above procedure is repeated, except that obtained during identification codes are compared with those already stored in the database, and in the case of coincidence of the codes, the person being identified is permitted to access or to pass.

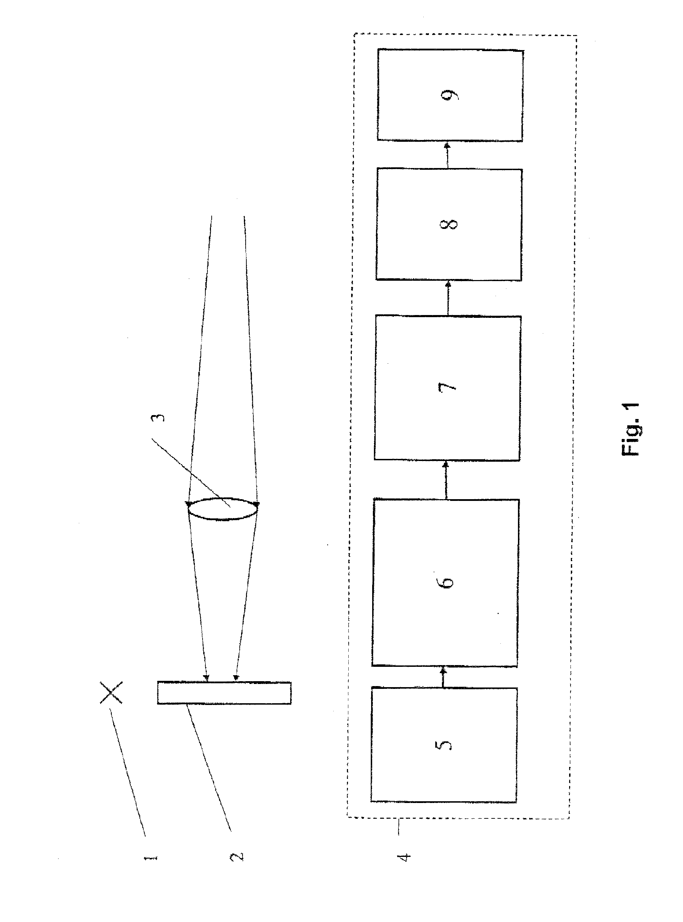

## Claims

1. The device for producing images of irises of the human eye comprising a means (1) for illuminating the iris of the eye in one or more spectral ranges which are close to the visible range, a means (2) for recording the signal reflected from the eyes and a means (4) for processing the images produced, said means comprising a unit (5) for detecting the centres of the pupils of the eyes on the image, a unit (6) for determining coordinates of the eyes relative to a set centre of the display and for constructing a straight reference line connecting the centres of the pupils, a unit (7) for processing the images of the iris of the eye in a normalized coordinate grid with segmentation and isolation of information fields, a unit (8) for generating codes, and a unit (9) for storing said codes.
